# EUROPEAN PATENT APPLICATION

(11) **EP 4 535 003 A1**
(43) Date of publication of application: **09.04.2025**
(21) Application number: 23811378.1
(22) Date of filing: 22.02.2023
(51) Int. Cl.: G01N 33/68, C07K 1/14, G01N 27/62, G01N 30/88

(54) **METHOD FOR PREPARING SAMPLE SOLUTION CONTAINING NEUROGRANIN-RELATED PEPTIDE, AND METHOD FOR ANALYZING NEUROGRANIN-RELATED PEPTIDE**

(30) Priority: 24.05.2022 JP 2022084497
(71) Applicant: SHIMADZU CORPORATION, Kyoto-shi, Kyoto 604-8511 (JP)
(72) Inventor: KANEKO, Naoki, Kyoto-shi, Kyoto 604-8511 (JP)
(74) Representative: Müller-Boré & Partner Patentanwälte PartG mbB
(86) International application number: PCT/JP2023/006501
(87) International publication number: WO 2023/228497

(57) **Abstract**

Provided is a method for analyzing a neurogranin-related peptide capable of suppressing variations in analysis results, and a method for preparing a biological sample containing a neurogranin-related peptide used therein. A method for preparing a sample solution containing a neurogranin-related peptide, the method includes mixing a biological sample containing a neurogranin-related peptide with an organic solvent having a relative polarity of 0.200 or more and 0.700 or less to prepare a sample solution having a final concentration of the organic solvent of 5.0 (v/v)% or more.

## Description

### TECHNICAL FIELD

The present invention relates to a method for preparing a sample solution containing a neurogranin-related peptide and a method for analyzing a neurogranin-related peptide.

### BACKGROUND ART

Alzheimer's disease is the leading type of dementia, and its afflicted people have increased more and more in recent years, and its research has become even more important. In the development of Alzheimer's disease, Aβ-related peptides such as amyloid β (AB) generated by cleavage of amyloid precursor protein (APP) are deeply involved. Then, it has been reported that a plurality of Aβ-related peptides in blood are detected by combining immunoprecipitation and mass spectrometry, and the detected specific Aβ-related peptide ratio can be effectively used as a blood biomarker of amyloid accumulation in the brain (Non-Patent Documents 1 and 2, Patent Documents 1 to 3).

On the other hand, Alzheimer's disease requires various biomarkers in order to monitor its disease progression, and there is a demand for biomarkers for reflecting each process of tau accumulation, neurodegeneration, and the like in addition to amyloid accumulation. Among them, neurogranin is one of biomarkers of neurodegeneration, and is reported to increase in cerebrospinal fluid (CSF) of Alzheimer's disease patients (Non-Patent Document 3 and Non-Patent Document 4). In addition, it has also been reported that fragmentation of neurogranin is promoted in the brain of Alzheimer's disease patients, and the fragment peptide is present in blood, and further, translational and modified neurogranin such as acetylation or glutathionation is also present (Non-Patent Document 5). Therefore, a means for confirming neurodegeneration by detecting a neurogranin-related peptide such as neurogranin or a fragment peptide thereof from a biological sample such as blood or CSF is expected.

### PRIOR ART DOCUMENTS

### PATENT DOCUMENTS

Patent Document 1: WO 2015/178398
Patent Document 2: WO 2017/47529
Patent Document 3: JP 2017-20980 A

### NON-PATENT DOCUMENTS

Non-Patent Document 1: Kaneko N, Nakamura A, Washimi Y, Kato T, Sakurai T, Arahata Y, Bundo M, Takeda A, Niida S, Ito K, Toba K, Tanaka K, Yanagisawa K.: Novel plasma biomarker surrogating cerebral amyloid deposition. Proc Jpn Acad Ser B Phys Biol Sci. 2014; 90(9): 353-364.
Non-Patent Document 2: Nakamura A, Kaneko N, Villemagne VL, Kato T, Doecke J, Dore V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K.: High performance plasma amyloid-β biomarkers for Alzheimer's disease. Nature. 2018; 554(7691): 249-254.
Non-Patent Document 3: Portelius E, Olsson B, Hoglund K, Cullen NC, Kvartsberg H, Andreasson U, Zetterberg H, Sandelius A, Shaw LM, Lee VMY, Irwin DJ, Grossman M, Weintraub D, Chen-Plotkin A, Wolk DA, McCluskey L, Elman L, McBride J, Toledo JB, Trojanowski JQ, Blennow K.: Cerebrospinal fluid neurogranin concentration in neurodegeneration: relation to clinical phenotypes and neuropathology. Acta Neuropathol. 2018; 136(3): 363-376.
Non-Patent Document 4: Thorsell A, Bjerke M, Gobom J, Brunhage E, Vanmechelen E, Andreasen N, Hansson O, Minthon L, Zetterberg H, Blennow K.: Neurogranin in cerebrospinal fluid as a marker of synaptic degeneration in Alzheimer's disease. Brain Res. 2010; 1362: 13-22.
Non-Patent Document 5: Kvartsberg H, Lashley T, Murray CE, Brinkmalm G, Cullen NC, Hoglund K, Zetterberg H, Blennow K, Portelius E.: The intact postsynaptic protein neurogranin is reduced in brain tissue from patients with familial and sporadic Alzheimer's disease. Acta Neuropathol. 2019; 137(1): 89-102.

### SUMMARY OF THE INVENTION

### PROBLEMS TO BE SOLVED BY THE INVENTION

Incidentally, as a biological sample for analyzing a neurogranin-related peptide, a blood sample is considered to be preferable from the viewpoint that it can be collected by a general test and that it is minimally invasive. However, since the amount of the neurogranin-related peptide present in blood or the like is very small, depending on the analysis method, a problem occurs in which a large variation occurs in the value of the analysis result even in the same blood sample.

An object of the present invention is to provide a method for analyzing a neurogranin-related peptide capable of suppressing variations in analysis results, and a method for preparing a biological sample containing a neurogranin-related peptide used therein.

### MEANS FOR SOLVING THE PROBLEMS

The preparation method of a first aspect of the present invention relates to a method for preparing a biological sample containing a neurogranin-related peptide, the method including: mixing a biological sample containing a neurogranin-related peptide with an organic solvent having a relative polarity of 0.200 or more and 0.700 or less to prepare a sample solution having a final concentration of the organic solvent of 5.0 (v/v)% or more.

The analysis method of a first aspect using the preparation method of the first aspect of the present invention relates to a method for analyzing a neurogranin-related peptide, the method including: a preparation step of performing the preparation method of the first aspect; and a measurement step of performing mass spectrometry, liquid chromatography, immunoassay, or surface plasmon resonance using the sample solution.

### EFFECTS OF THE INVENTION

According to the preparation method of the first aspect of the present invention, degradation of a neurogranin-related peptide contained in a biological sample can be suppressed. According to the analysis method of the first aspect of the present invention, variation of the analysis results of the neurogranin-related peptide can be suppressed.

### BRIEF DESCRIPTION OF THE DRAWINGS

FIG. 1 is a mass spectrum (final concentration: 0 to 5%) for each DMSO concentration in a sample solution containing an Ng-related peptide. The vertical axis represents relative intensity, and the horizontal axis represents m/z.
FIG. 2 is a mass spectrum (concentration: 0 to 30%) for each DMSO concentration in the sample solution containing an Ng-related peptide.
FIG. 3 is a graph (final concentration: 0 to 5%) showing the relationship between a ratio of SIL-Ng50-75 to SIL-Ng50-78 and the DMSO concentration. The vertical axis represents the ratio of SIL-Ng50-75 to SIL-Ng50-78, and the horizontal axis represents the final concentration of DMSO.
FIG. 4 is a graph (final concentration: 0 to 30%) showing the relationship between the ratio of SIL-Ng50-75 to SIL-Ng50-78 and the DMSO concentration.
FIG. 5 is a graph (final concentration: 0 to 5%) showing the relationship between a ratio of rNg1-75 to rNg1-78 and the DMSO concentration. The vertical axis represents the ratio of rNg1-75 to rNg1-78, and the horizontal axis represents the final concentration of DMSO.
FIG. 6 is a graph (final concentration: 0 to 30%) showing the relationship between a ratio of rNg1-75 to rNg1-78 and the DMSO concentration.
FIG. 7 is a mass spectrum for each incubation time in a sample solution containing an Ng-related peptide as a comparative example. The vertical axis represents relative intensity, and the horizontal axis represents m/z.
FIG. 8 is a graph showing the relationship between the ratio of SIL-Ng50-75 to SIL-Ng50-78 and the incubation time in Comparative Example. The vertical axis represents the ratio of SIL-Ng50-75 to SIL-Ng50-78, and the horizontal axis represents the room temperature incubation time.
FIG. 9 is a graph showing the relationship between the ratio of rNg1-75 to rNg1-78 and the incubation time in comparative example. The vertical axis represents the ratio of rNg1-75 to rNg1-78, and the horizontal axis represents the incubation time at room temperature.
FIG. 10 is a graph showing the relationship between the ratio of SIL-Ng50-75 to SIL-Ng50-78 and various solvents. The vertical axis represents the ratio of SIL-Ng50-75 to SIL-Ng50-78, and the horizontal axis represents various solvents (final concentration: 10%).
FIG. 11 is a graph showing the relationship between the ratio of rNg1-75 to rNg1-78 and various solvents. The vertical axis represents the ratio of rNg1-75 to rNg1-78, and the horizontal axis represents various solvents (final concentration: 10%).
FIG. 12 is a mass spectrum (final concentration: 0 to 20%) for each ACN concentration in a sample solution containing an Ng-related peptide. The vertical axis represents relative intensity, and the horizontal axis represents m/z.
FIG. 13 is an enlarged view in the vicinity of m/z 2150 to 2540 in FIG. 12.
FIG. 14 is an enlarged view in the vicinity of m/z 4800 to 5000 in FIG. 12.

### MODE FOR CARRYING OUT THE INVENTION

### 1. First embodiment

### 1-1. Preparation method

In the method for preparing a sample solution containing a neurogranin-related peptide according to a first embodiment, a biological sample and an organic solvent having a relative polarity of 0.200 or more and 0.700 or less are mixed.

The "neurogranin-related peptide" (hereinafter, abbreviated as "Ng-related peptide") includes neurogranin, translated/modified neurogranin, and fragment peptides thereof.

The biological sample is a sample containing an Ng-related peptide, and examples thereof include body fluids such as blood, cerebrospinal fluid, urine, body secretion, saliva, sputum, and feces. The blood includes whole blood, plasma, serum, and the like. The blood may be obtained by subjecting whole blood collected from an individual to treatment such as centrifugation and cryopreservation. Preferably blood is exemplified. Blood is less invasive than cerebral bone marrow fluid, and is a target sample for screening in a medical examination or the like, and is easily available.

The organic solvent has a relative polarity of 0.200 or more and 0.700 or less. The lower limit of the relative polarity is preferably 0.300 or more, more preferably 0.370 or more, and most preferably 0.400 or more, and the upper limit is preferably 0.600 or less, more preferably 0.500 or less, and most preferably 0.450 or less. The relative polarity is disclosed in detail, for example, in Solvents and Solvent Effects in Organic Chemistry", Christian Reichards, Wiley-VCH Publishers, 3rd ed., 2003.

Examples of such an organic solvent include acetone (0.355), dimethylformamide (0.386), dimethylsulfoxide (0.444), acetonitrile (0.460), 2-propanol (0.546), ethanol (0.654), 1-butanol (0.586), and 2-butanol (0.506). The number in parentheses indicates the value of the relative polarity. These may be used singly or in combination of two or more kinds thereof. From the viewpoint that the degradation of the Ng-related peptide can be more reliably suppressed, dimethylsulfoxide (DMSO; dimethyl sulfoxide). On the other hand, from the viewpoint that the Ng-related peptide can be detected with even higher sensitivity in addition to the suppression of the decomposition of the Ng-related peptide, acetonitrile (ACN) is preferably used.

In the preparation step, the biological sample and the organic solvent are mixed so that the final concentration of the organic solvent is 5.0 (v/v)% or more with respect to the prepared sample solution containing an Ng-related peptide. The lower limit of the final concentration is preferably 10.0 (v/v)% or more, and the upper limit is, for example, 30.0 (v/v)% or less and preferably 20.0 (v/v)% or less. By setting the concentration to the above lower limit or more, the suppression of the decomposition of the Ng-related peptide by mixing with the organic solvent is effectively exhibited. In addition, by setting the concentration to the above upper limit or less, it is possible to suppress a decrease in the detection sensitivity of the Ng-related peptide due to excessive addition of the organic solvent. Furthermore, when acetonitrile is used, it is more preferably 15.0 (v/v)% or more and 20.0 (v/v)% or less in addition to the above range. By setting these concentration ranges, Ng-related peptides can be detected with higher sensitivity, and thus, for example, peaks of more types of Ng-related peptides can be detected in a mass spectrum obtained by the mass spectrometry described later. In addition, the addition amount of the organic solvent with respect to 100 parts by volume of the biological sample is, for example, 5.0 parts by volume or more, preferably 10.0 parts by volume or more, and is, for example, 200 parts by volume or less, and preferably 50.0 parts by volume or less.

In the preparation step, it is preferable to add a buffer as necessary in order to achieve the above final concentration. Examples of the buffer include a Tris buffer, a phosphate buffer, a HEPES buffer, and an ammonium acetate buffer. As a result, the pH of the sample solution can be made neutral to achieve the final concentration, and the sample solution can be easily purified in the purification step described later. The pH of the buffer is preferably neutral, for example, pH 6.0 or more and preferably 6.5 or more, and is, for example, 8.5 or less and preferably 8.0 or less. A mixing ratio of the buffer is, for example, 5.0 (v/v)% or more and preferably 10.0 (v/v)% or more, and is, for example, 80.0 (v/v)% or less and preferably 40.0 (v/v)% or less with respect to the prepared sample solution containing an Ng-related peptide. In addition, the amount of the buffer with respect to 100 parts by volume of the organic solvent is 50.0 parts by volume or more and preferably 100 parts by volume or more, and is, for example, 1000 parts by volume or less and preferably 500 parts by volume or less.

When the purification step described later is continuously performed, a surfactant may be mixed with the biological sample together with the buffer in a first binding step as in a binding solution described later.

Thus, a sample solution containing an Ng-related peptide is prepared (produced). Since this sample solution contains a specific amount of a specific organic solvent, it is possible to suppress the degradation of the Ng-related peptide in the sample solution. That is, even if the Ng-related peptide is stored for a predetermined time, the temporal decrease in the Ng-related peptide content is suppressed, and the amount of the Ng-related peptide after biological sample collection is hardly changed and is almost constant. Therefore, even if the measurement (described later) is performed after the storage for a predetermined time, the change in the detected concentration due to the storage time is suppressed, and the variation in the detected concentration can be suppressed. In addition, the degradation caused in the measurement step can be suppressed. Therefore, it is possible to suppress the change of the detected concentration due to the time in the measurement step, and to suppress the variation in the detected concentration.

The present inventor has focused on an Ng-related peptide contained in a biological sample such as blood collected from a living body, and has found that the Ng-related peptide is degraded (digested) over time in the biological sample, specifically, the Ng-related peptide is cleaved at the C-terminus of the 75th amino acid of neurogranin over time by a protease in the biological sample. That is, taking Ng1-78 as an example of an analysis target, when a blood sample is collected (separated) from a living body and analyzed, it has been found that Ng1-78 is gradually degraded into Ng1-75 and Ng76-78 during storage time or analysis preparation time from collection to analysis, and Ng1-78 changes (decreases) according to the time. Therefore, as a result of further intensive studies, it was found that when a specific amount of a specific organic solvent is present in a biological sample, degradation of the amino acid is suppressed, and the preparation method of the present invention has been completed. Note that Ng1-78 is always degraded also in the living body, but Ng1-78 is produced from the living body, and thus Ng1-78 is kept constant in the living body, and the above-described decrease occurs from the time when the biological sample is separated from the living body and used for analysis.

### 1-2. Analysis step

An analysis method according to a first embodiment is a method for analyzing an Ng-related peptide in a biological sample, and includes a preparation step and a measurement step in order. The preparation step is the preparation method described above, and the measurement step is performed by mass spectrometry. As a preferred embodiment, a purification step is performed in the measurement step. That is, the measurement step sequentially includes a purification step of purifying the Ng-related peptide in the sample solution containing an Ng-related peptide and a detection step of performing the mass spectrometry. Hereinafter, this mode will be described in detail.

### [Purification step]

The purification step is preferably affinity purification. The affinity purification may be performed once or twice, but the purification is preferably performed twice from the viewpoint of being able to detect the Ng-related peptide with higher sensitivity. In this case, the purification step includes a first binding step (an example of a binding step), a first washing step (an example of a washing step), a first elution step, a neutralization step, a second binding step, a second washing step, and a second elution step (an example of an elution step) in this order. Hereinafter, each step will be described in detail.

### (First binding step)

In the first binding step, the sample solution containing an Ng-related peptide obtained in the preparation step is brought into contact with a first carrier. As a result, the Ng-related peptide in the sample solution is bound to the first carrier, and thus a first conjugate is obtained.

The first carrier may be any carrier to which the Ng-related peptide can be bound, and examples thereof include an antibody-immobilizing carrier.

The antibody immobilized on the first carrier is an antibody having an antigen binding site capable of recognizing an Ng-related peptide (anti-Ng-related peptide antibody), and examples thereof include an immunoglobulin having an antigen binding site capable of recognizing an Ng-related peptide or a fragment thereof.

Examples of the immunoglobulin include IgG (IgG1, IgG2, IgG3, IgG4), IgM, IgA, IgY, IgD, and IgE. Examples of the immunoglobulin fragment include F(ab')2, F(ab'), F(ab), Fd, Fv, L chain, and H chain. More specifically, clones NG2, NG7, EPR21152, fragments thereof, and the like. The antibody may be either a monoclonal antibody or a polyclonal antibody.

Examples of the material of the first carrier include agarose, sepharose, dextran, silica gel, polyacrylamide, polystyrene, polyethylene, polypropylene, polyester, polyacrylonitrile, a (meth)acrylic acid-based polymer, a fluororesin, a metal complex resin, glass, metal, and a magnetic body.

The shape of the first carrier may be any shape such as a spherical shape (including a bead shape), a plate shape, a needle shape, and an irregular shape, and may be a flow path wall in a microdevice or the like.

At this time, in addition to the first carrier, a binding solution may be further added as necessary. The binding solution is preferably a neutral buffer containing a surfactant. Examples of the buffer include those similar to the buffer described above in the preparation step. The pH of the binding solution is, for example, pH 6.0 or more and preferably 6.5 or more, and is, for example, 8.5 or less and preferably 8.0 or less.

Examples of the surfactant contained in the binding solution include a neutral surfactant having a hydrophobic group having 7 or more and 15 or less carbon atoms (preferably, 9 or more and 11 or less). This makes it possible to suppress non-specific adsorption to the first conjugate and to reduce ionization interference in mass spectrometry. Examples of such surfactants include surfactants having maltose in a hydrophilic moiety, such as n-nonyl-β-D-maltoside, n-nonyl-β-D-thiomaltoside, n-decyl-β-D-maltoside, and n-undecyl-β-D-maltoside (UDM); surfactants having trehalose in a hydrophilic moiety, such as α-D-glucopyranosyl α-D-glucopyranoside monodecanoate (trehalose C10); and surfactants having glucose in a hydrophilic moiety, such as n-decyl-β-D-glucoside. These surfactants can be used singly or in combination of two or more kinds thereof.

The surfactant concentration in the binding solution is, for example, 0.01% (w/v) or more and preferably 0.05% (w/v) or more, and is, for example, 10% (w/v) or less and preferably 3% (w/v) or less. When the surfactant concentration is within the above range, micelles are sufficiently formed, so that the effect of the surfactant can be reliably exhibited.

Before the first binding step, pretreatment for removing antibodies such as IgG and IgM may be performed as necessary.

### (First washing step)

In the first washing step, after the first binding step, the first conjugate is washed using a first washing solution.

The first washing solution is preferably a neutral buffer containing a surfactant. This makes it possible to effectively remove highly hydrophobic undesirable components (blood proteins, lipids, glycolipids, and the like). Examples of the neutral buffer and the surfactant in the first washing solution include those similar to the neutral buffer and the surfactant exemplified in the binding solution.

The surfactant concentration in the first washing solution is, for example, 0.01% (w/v) or more and preferably 0.02% (w/v) or more, and is, for example, 5% (w/v) or less and preferably 2% (w/v) or less. When the surfactant concentration is within the above range, micelles are sufficiently formed, so that the effect of the surfactant can be reliably exhibited.

As a washing method, a known method may be adopted, and washing is preferably performed a plurality of times. For example, washing is performed using a neutral buffer containing a surfactant, followed by washing using the neutral buffer not containing the surfactant.

As a neutral buffer containing no surfactant, a neutral buffer similar to the neutral buffer exemplified in the binding solution can be used. This makes it possible to suppress foaming caused by the surfactant remaining in the first conjugate.

As the washing method, a general method may be employed, and examples thereof include a method for stirring a carrier in a washing solution, a method for spraying a washing solution from a washing nozzle, and the like. After washing with these neutral buffers, washing with water may be further performed as necessary.

### (First elution step)

In the first elution step, the first conjugate is brought into contact with a first acidic solution after the first washing step. As a result, the Ng-related peptide is dissociated from the first conjugate, and the Ng-related peptide is eluted into the first acidic solution. As a result, a first eluate containing the Ng-related peptide is obtained.

Examples of the first acidic solution include an acidic aqueous solution such as a glycine buffer and hydrochloric acid, and preferably include a glycine buffer. The pH of the first acidic solution is, for example, 3.5 or less and preferably 3.0 or less, and is, for example, 0.5 or more and preferably 1.0 or more.

The first acidic solution preferably contains a surfactant. This makes it possible to more reliably dissociate the Ng-related peptide from the first conjugate. In addition, the eluted Ng-related peptide is inhibited from adhering to a container such as a test tube or a microplate. Therefore, the recovery rate of the Ng-related peptide can be reliably improved, and the detection sensitivity can be improved. Examples of the surfactant used in the first acidic solution include the same surfactants as those exemplified in the binding solution. The surfactant concentration in the first acidic solution is the same as the surfactant concentration in the first washing solution.

### (Neutralization step)

In the neutralization step, after the first elution step, the first eluate is mixed with a neutral buffer. As a result, the first eluate is neutralized to obtain a purified solution containing an Ng-related peptide.

The neutral buffer used in the neutralization step preferably contains a surfactant. This makes it possible to suppress non-specific adsorption to a second conjugate in a second binding step. Examples of the neutral buffer and the surfactant in the neutralization step include those similar to the neutral buffer and the surfactant exemplified in the binding solution. The surfactant concentration in the neutral buffer is the same as the surfactant concentration in the first binding solution.

The pH of the obtained purified solution is neutral, for example, pH 6.0 or more and preferably 6.5 or more, and is, for example, 8.5 or less and preferably 8.0 or less. Accordingly, in the second binding step, the binding efficiency can be improved.

### (Second binding step)

In the second binding step, the purified solution is brought into contact with a second carrier after the neutralization step. As a result, the Ng-related peptide in the purified solution is bound to the second carrier to obtain a second conjugate.

The second carrier is preferably an antibody-immobilizing carrier, and specific examples thereof include the same antibody-immobilizing carriers as exemplified for the first carrier.

### (Second washing step)

In the second washing step, after the second binding step, the second conjugate is washed using the second washing solution.

The second washing solution is preferably a neutral buffer containing a surfactant. As a result, for example, this makes it possible to effectively remove highly hydrophobic undesirable components (blood proteins, lipids, glycolipids, and the like). Examples of the neutral buffer and the surfactant using the second washing solution include those similar to the neutral buffer and the surfactant exemplified in the binding solution. The surfactant concentration in the second washing solution is the same as the surfactant concentration in the first washing solution.

As the washing method, a known method may be adopted, and specifically, a method similar to the washing method exemplified in the first washing step may be performed.

### (Second elution step)

In the second elution step, the second conjugate is brought into contact with the second acidic solution after the second washing step. As a result, the Ng-related peptide is dissociated from the second conjugate, and the Ng-related peptide is eluted into the second acidic solution. As a result, a second eluate containing the Ng-related peptide is obtained.

Examples of the acidic aqueous solution constituting the second acidic solution include those similar to the first acidic solution exemplified in the first elution step, and preferably include hydrochloric acid.

The second acidic solution preferably contains a volatile organic solvent. As a result, the Ng-related peptide can be efficiently dissociated from the second conjugate and eluted in the second acidic solution, and the recovery rate of the Ng-related peptide can be improved.

Examples of the volatile organic solvent include organic solvents miscible with water in an optional ratio, and examples thereof include acetonitrile, methanol, ethanol, acetone, toluene, isopropanol, hexane, butanol, cyclohexane, ethylene glycol, benzene, chloroform, acetaldehyde, triethylamine, phenol, naphthalene, formaldehyde, tetrahydrofuran, and ethyl acetate, and preferred examples thereof include acetonitrile, methanol, ethanol, acetone, and isopropanol. These organic solvents can be used singly or in combination of two or more kinds thereof.

The concentration of the volatile organic solvent in the second acidic solution is, for example, 10% (v/v) or more and preferably 25% (v/v) or more, and is, for example, 90% (v/v) or less and preferably 80% (v/v) or less. When the concentration is within the above range, the Ng-related peptide can be efficiently dissociated from the second carrier, and the sensitivity (S/N ratio) at the time of mass spectrometry can be improved.

The second acidic solution preferably further contains amino acid such as methiotin. As a result, the oxidation of the Ng-related peptide can be reduced until the analysis is started after the Ng-related peptide is placed in the mass spectrometer, and the detection sensitivity can be improved. The amino acid concentration in the second acidic solution is, for example, 0.01 mM or more and preferably 0.05 mM or more, and is, for example, 5 mM or less and preferably 1 mM or less.

The second acidic solution preferably further contains a protein of 9 kDa or more. As a result, since the protein is contained in a mass spectrometry sample, the protein acts as a proton receptor at the time of mass spectrometry, and the Ng-related peptide can be efficiently ionized, and as a result, the detection sensitivity of the Ng-related peptide can be remarkably improved. The upper limit of the mass of the protein is, for example, 100 kDa or less, preferably 15 kDa or less. The protein is not limited as long as it has a mass of 9 kDa or more, and specifically includes bovine serum albumin (BSA), cytochrome, ovalbumin, lysozyme, and the like. The concentration of the protein in the second acidic solution is, for example, 10 nM or more and preferably 30 nM or more, and for example, 600 nM or less and preferably 150 nM or less.

### [Detection step]

In the detection step, after the purification step, the second eluate is subjected to the mass spectrometry to detect the Ng-related peptide.

Examples of the ionization method in the mass spectrometry include MALDI (Matrix Assisted Laser Desorption/Ionization) and ESI (Electrospray Ionization), and APCI (Atmospheric Pressure Chemical Ionization). MALDI is preferable from the viewpoint that a trace amount of Ng-related peptide in the sample solution can be detected with high sensitivity.

Examples of the mass spectrometry include, but are not limited to, TOF-MS (time-of-flight mass spectrometry), IT-MS (ion trap mass spectrometry), IT-TOF-MS (ion trap-time-of-flight mass spectrometry), and FTICR-MS (Fourier transform ion cyclotron mass spectrometry). When MALDI is used as the ionization method, these mass spectrometries are collectively referred to as MALDI-MS.

As a specific detection operation by MALDI-MS, for example, first, a matrix-containing solution is dropped onto a MALDI plate and dried (crystallized) to arrange the matrix. Subsequently, the second eluate is dropped and dried on the matrix to obtain a MALDI-MS sample. Subsequently, the MALDI-MS sample is irradiated with a laser to ionize the Ng-related peptide, and the ionized peptide is detected by a detector by the above apparatus and output as a mass spectrum. Then, by analyzing the mass spectrum, an analysis result such as the type and concentration of the Ng-related peptide contained in the biological sample can be obtained.

Examples of the matrix include α-cyano-4-hydroxycinnamic acid (CHCA), 2,5-dihydroxybenzoic acid, sinapinic acid, 3-aminoquinoline, and the like. These matrices can be used singly or in combination of two or more kinds thereof.

Examples of the solvent containing the matrix include acetonitrile, trifluoroacetic acid, methanol, ethanol, and water. These solvents can be used singly or in combination of two or more kinds thereof. The matrix concentration in the matrix-containing solution is, for example, 0.1 mg/mL or more and preferably 0.5 mg/mL or more, and is, for example, 50 mg/mL or less and preferably 10 mg/mL or less.

Preferably, a matrix additive is used in combination with the matrix. Examples of the matrix additive include a phosphonic acid group-containing compound and an ammonium salt, and preferably include a phosphonic acid group -containing compound from the viewpoint of being able to suppress an adverse effect on the background due to the remaining of the washing solution. Examples of the phosphonic acid group-containing compound include phosphonic acid, methylphosphonic acid, phenylphosphonic acid, 1-naphthylmethylphosphonic acid, methylenediphosphonic acid (MDPNA), ethylenediphosphonic acid, ethane-1-hydroxy-1,1-diphosphonic acid, nitrilotriphosphonic acid, and ethylene diaminotetraphosphonic acid. The matrix additive concentration in the matrix-containing solution is, for example, 0.01% (w/v) or more and preferably 0.1% (w/v) or more, and is, for example, 10% (w/v) or less and preferably 1% (w/v) or less.

According to this analysis method, it is possible to suppress variations in analysis results of Ng-related peptides. In particular, even when a biological sample collected from a living body is stored for a desired time, it is possible to suppress variations (for example, variations in peak intensities and ratios of various Ng-related peptides in a mass spectrum, concentrations calculated from the peak intensities and ratios, and the like) in measured values for each storage time.

As a result of further intensive studies based on the findings of the preparation method described above, the present inventor has found that during an Ng-related peptide analysis, by using, as a measurement sample for analysis, a solution in which a specific organic solvent is previously present in a biological sample in a specific amount to suppress degradation of the Ng-related peptide, it is possible to suppress variations in Ng-peptide analysis caused by storage time or analysis preparation time. And the analysis method of the present invention has been completed.

### 1-3. Modification

In the above embodiment, the mass spectrometry is performed as the detection step, but for example, liquid chromatography, immunoassay, or surface plasmon resonance may be performed as the detection step. These measurement methods may be performed according to a known or usual method except that the Ng-containing sample solution prepared in the preparation step is used as the measurement sample. From the viewpoint that even a minute amount of Ng-related peptide in blood can be reliably detected, it is preferable to perform the mass spectrometry as the detection step.

### 2. Aspects

It is understood by those skilled in the art that the plurality of exemplary embodiments described above are specific examples of the following aspects.

(Aspect 1) A method for preparing a sample solution containing a neurogranin-related peptide according to one embodiment, the method may include mixing a biological sample containing a neurogranin-related peptide with an organic solvent having a relative polarity of 0.200 or more and 0.700 or less to prepare a sample solution having a final concentration of the organic solvent of 5.0 (v/v)% or more.

(Aspect 2) In the preparation method according to Aspect 1, the final concentration of the organic solvent may be 5.0 (v/v)% or more and 20.0 (v/v)% or less.

(Aspect 3) In the preparation method according to Aspect 1 or 2, the biological sample may be blood.

(Aspect 4) In the preparation method according to any one of Aspect 1 to 3, a buffer solution may be further mixed with the biological sample.

(Aspect 5) In the preparation method according to any one of Aspects 1 to 4, the organic solvent may be dimethyl sulfoxide.

(Aspect 6) In the preparation method according to any one of Aspects 1 to 4, the organic solvent may be acetonitrile.

(Aspect 7) A method for analyzing a neurogranin-related peptide according to one embodiment of the present invention, the method may include a preparation step of performing the preparation method according to any one of Aspects 1 to 6; and a measurement step of performing mass spectrometry, liquid chromatography, immunoassay, or surface plasmon resonance using the sample solution.

(Aspect 8) In the analysis method according to Aspect 7, the measurement step may include (a) a binding step of bringing the sample solution into contact with a carrier to obtain a conjugate in which the neurogranin-related peptide is bound to the carrier; (b) a washing step of washing the conjugate using a washing solution; (c) an elution step of bringing the conjugate into contact with an acidic solution to obtain an eluate in which the neurogranin-related peptide is eluted in the acidic solution; and (d) a detection step of detecting the neurogranin-related peptide in the eluate by mass spectrometry.

(Aspect 9) In the analysis method according to Aspect 8, the ionization method of the mass spectrometry may be a matrix-assisted laser desorption/ionization method.

### EXAMPLES

Next, the present invention will be described in detail with reference to Examples, but the scope of the present invention is not limited thereto.

### <Example 1>

### [Preparation step]

50 µL of DMSO (dimethyl sulfoxide) was mixed with 200 µL of a buffer containing a surfactant (0.1% n-undecyl-β-D-maltoside (UDM), 800 mM GlcNAc, 100 mM Tris-HCl, 300 mM NaCl; pH 7.4) to prepare a DMSO-containing buffer having a DMSO concentration of 20.0 (v/v)%. This buffer was subsequently mixed with stable isotope labelled Ng50-78 (SIL-Ng50-78; manufactured by Toray Research Center Inc.) of 50 pM, and His-tagged recombinantNg (rNg; manufactured by Abcam) of 6 nM to prepare an Ng-DMSO-containing buffer. In SIL-Ng50-78, the carbon atoms and nitrogen atoms of Pro and Val are substituted with ¹³C and ¹⁵N, respectively.

Next, 250 µL of a commercially available human plasma sample was mixed with 250 µL of the Ng-DMSO-containing buffer. Thus, a sample solution containing Ng-related peptides was prepared in which the final concentration of DMSO was 10.0 (v/v)%. This sample solution containing the Ng-related peptides was incubated at room temperature for 3 hours.

### [Purification step]

### (First binding step, first washing step, and first elution step)

A clone NG2 (manufactured by BioLegend, Inc.) of an anti-Ng antibody (IgG1) having 52-63 residues of human Neurogranin (Ng) as an epitope was prepared. To 100 µg of an anti-Ng antibody, 5.5 mg of magnetic beads (Dynabeads M-270 Epoxy) were added in an immobilization buffer (0.1 M phosphate buffer containing 1.5 M ammonium sulfate; pH 7.4) at 37°C for 16 to 24 hours. Thus, antibody beads which are antibody-immobilizing carriers were produced.

The incubated sample solution containing the Ng-related peptides was allowed to stand on ice for 5 to 60 minutes, then the antibody beads were mixed and shaken on ice or at 4°C for 1 hour. Thereafter, the antibody beads were washed once or three times with 100 µL of the first washing buffer (0.05% UDM, 50 mM Tris-HCl, 150 mM NaCl; pH 7.4) and washed once or twice with 50 µL of 50 mM ammonium acetate buffer. Thereafter, the antibody beads were brought into contact with the first acidic solution (0.05% UDM, 50 mM glycine buffer; pH 2.8) to elute the Ng-related peptide into the first acidic solution. Thus, a first eluate containing the Ng-related peptide was obtained.

### (Neutralization step)

The first eluate was mixed with a neutral buffer (0.1% UDM, 800 mM GlcNAc, 300 mM Tris-HCl, 300 mM NaCl; pH 7.4) to give a purified solution.

### (Second binding step, second washing step, and second elution step)

The purified solution was mixed with antibody beads and shaken on ice or at 4°C for 1 hour. Thereafter, the antibody beads were washed twice or five times with 50 µL of the second washing buffer (0.05% UDM, 50 mM Tris-HCl, 150 mM NaCl; pH 7.4) and once or twice with 50 µL of 50 mM ammonium acetate buffer, and washed once with 30 µL of water. Thereafter, the antibody beads were brought into contact with a second acidic solution (70% (v/v) acetonitrile aqueous solution containing 5 mM hydrochloric acid and 0.1 mM methionine, and 50 nM BSA) to elute the Ng-related peptide into the second acidic solution. Thus, a second eluate containing the Ng-related peptide was obtained.

### [Detection step]

AXIMA Performance (Shimadzu/KRATOS, Manchester, UK), which is a MALDI-TOF MS apparatus, was used as a mass spectrometer. Using α-cyano-4-hydroxycinnamic acid (CHCA) as a matrix for Linear TOF, methylene diphosphonic acid (MDPNA) as a matrix additive, and acetonitrile as a solvent, a 2 mg/mL CHCA/0.2% (w/v) MDPNA matrix solution was prepared. 0.5 µL each of the matrix solution was added dropwise to 4 wells of a MALDI plate (µFocus MALDI plate 900 µm (Hudson Surface Technology, Inc., Fort Lee, NJ)) and dried, and then 1 µL each of the second eluate was further added dropwise thereto and dried.

Then, MALDI-TOF MS was then activated to detect Ng-related peptides (SIL-Ng50-78, SIL-Ng50-75, rNg1-78, and rNg1-75). As a setting condition, mass spectrum data was acquired by Linear TOF in a positive ion mode. 400 spots and 16000 shots were accumulated for 1well. Linear TOF m/z values are shown as average mass of peaks. The m/z value was calibrated using human angiotensin II and human ACTH fragment 18-39, bovine insulin oxidized beta-chain, bovine insulin, and cytochrome c as external standards.

When Ng50-78 and Ng50-75 that were not stably isotope-labeled, and Ng1-78 and Ng1-75 that were not recombinant were used as targets, Ng50-78, Ng50-75, Ng1-78, and Ng1-75 that were originally present in plasma could not be distinguished from each other, and the degradation amount could not be accurately evaluated. Therefore, stably isotope-labeled Ng50-78 and Ng50-75, and His-tagged rNg1-78 and rNg1-75 that were not present in human plasma were used as targets for detection.

Table 1 shows the Ng-related peptides detected by the mass spectrum. In the table, * represents rNg1-75 and rNg1-78 represent the theoretical average m/z of the divalent ion peak.

**[Table 1]**

| Peptide Name | Sequence | Theoretical average m/z | SEQ No. |
|---|---|---|---|
| SIL-Ng50-75 | ERGRKGPGPGGPGGAGVARGGAGGGP | 2217.5 | 1 |
| SIL-Ng50-78 | ERGRKGPGPGGPGGAGVARGGAGGGPSGD | 2476.7 | 2 |
| rNg1-75 | GSSHHHHHHSSGLVPRGSHMGSMDCCTENACSKPDDDILDIPLDDPGAN AAAAKIQASFRGHMARKKIKSGERGRKGPGPGGPGGAGVARGGAGGGP | 4834.4 * | 3 |
| rNg1-78 | GSSHHHHHHSSGLVPRGSHMGSMDCCTENACSKPDDDILDIPLDDPGAN AAAAKIQASFRGHMARKKIKSGERGRKGPGPGGPGGAGVARGGAGGGPSGD | 4963.0 * | 4 |

### [Change of final concentration]

The blending amounts of DMSO and the buffer were changed, and the DMSO concentration of the sample solution containing the Ng-related peptides obtained in the preparation step was changed to 0 to 25.0 (v/v%) (specifically, 0%, 0.05%, 0.25%, 0.5%, 2.5%, 5.0%, 10.0%, 15.0%, 20.0%, and 25.0%). Otherwise, the preparation step to the detection step were performed in the same manner as described above.

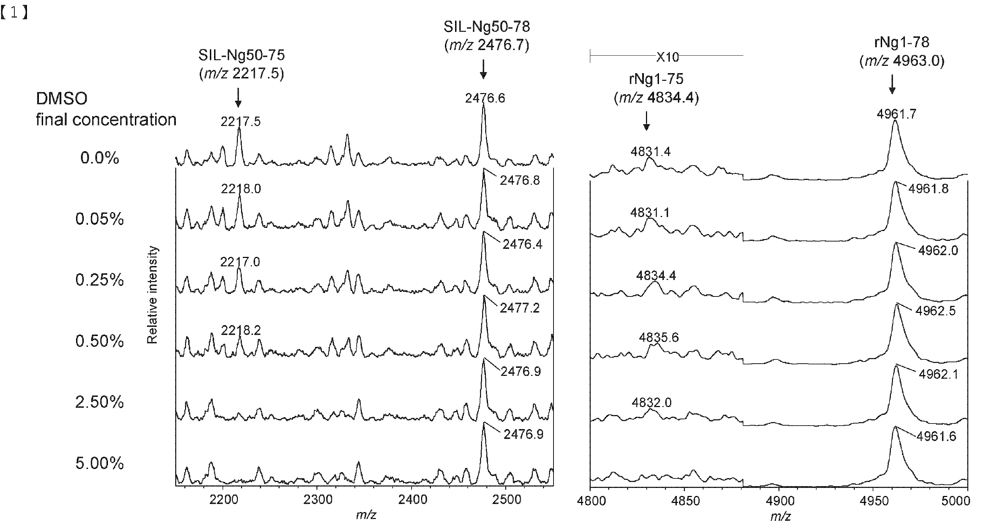

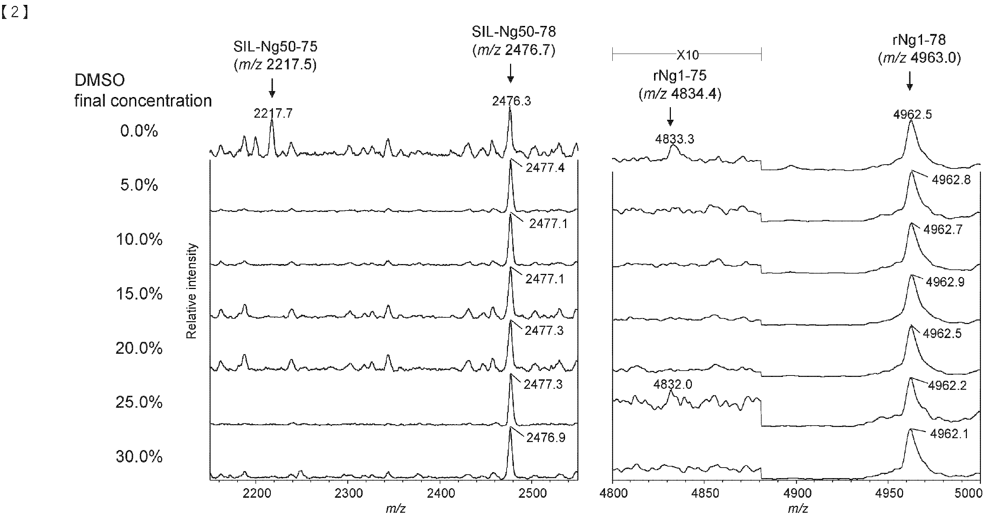

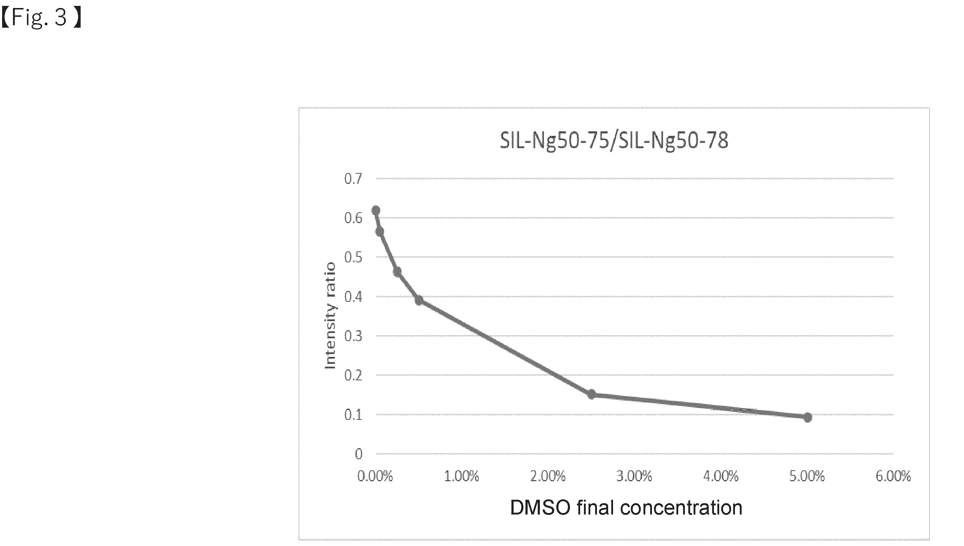

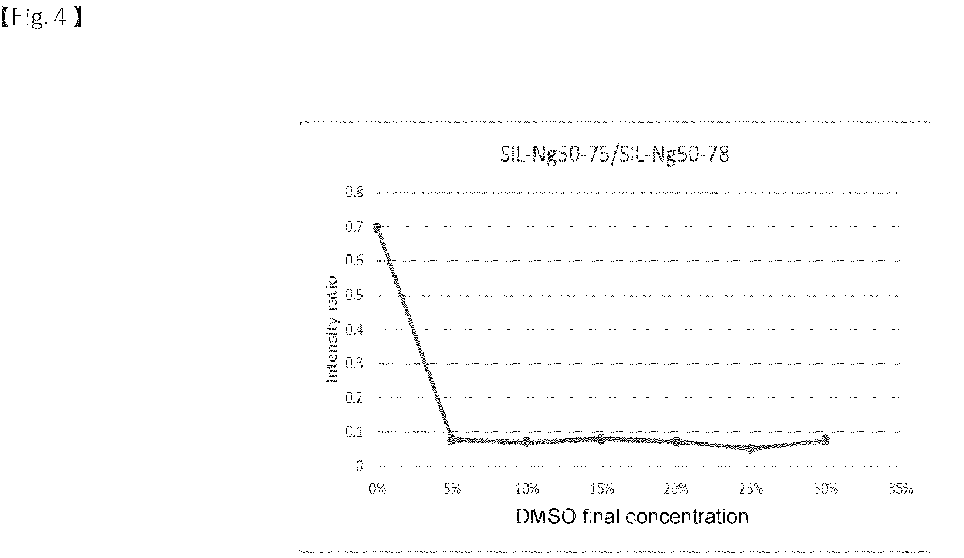

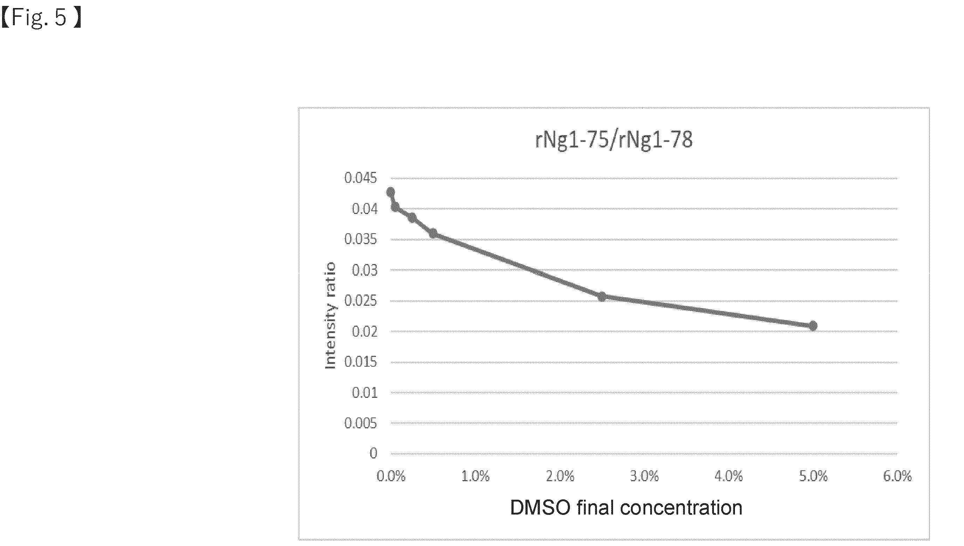

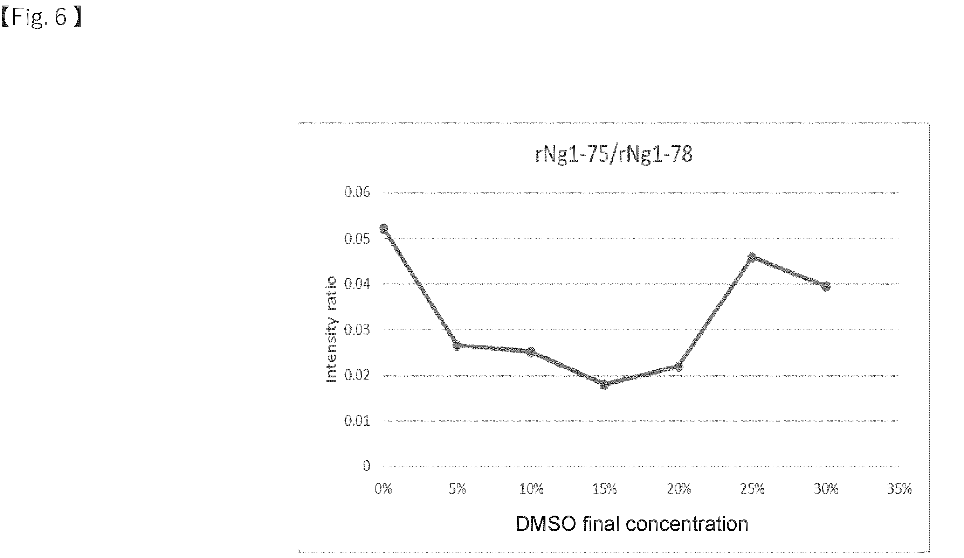

These results are shown in FIGS. 1 to 6. Specifically, mass spectra for each DMSO concentration in the sample solution containing Ng-related peptides are shown in FIG. 1 (final concentration: 0 to 5%) and FIG. 2 (final concentration: 0 to 30%), and graphs showing the relationship between the intensity ratio of the degraded peptide to the peptide to be degraded and the DMSO concentration are shown in FIGS. 3 and 4 (SIL-Ng50-75/SIL-Ng50-78) and FIGS. 5 and 6 (rNg1-75/rNg1-78).

### <Comparative Example 1>

In the preparation step, the preparation step to the detection step were performed in the same manner as described above except that DMSO was not mixed (that is, the DMSO concentration is set to 0%) and the incubation time was set to 0 minute, 1 hour, and 3 hours, respectively.

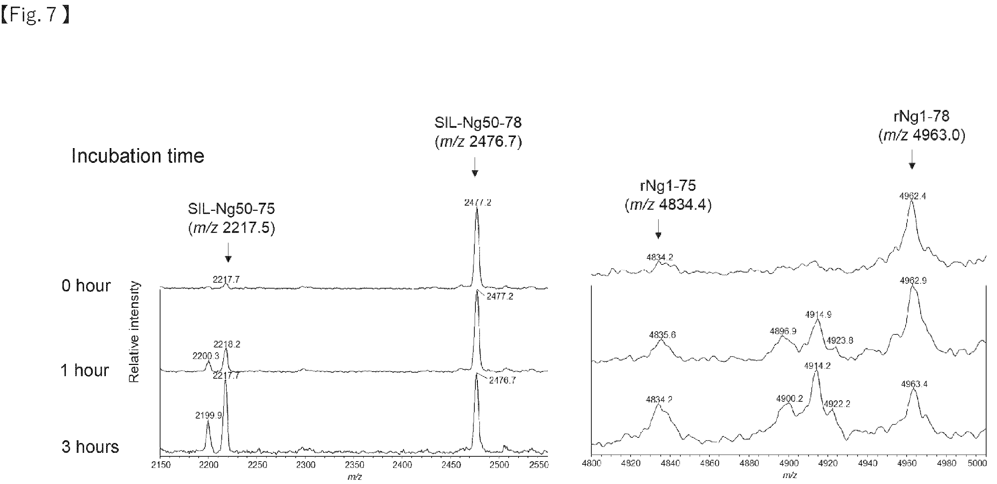

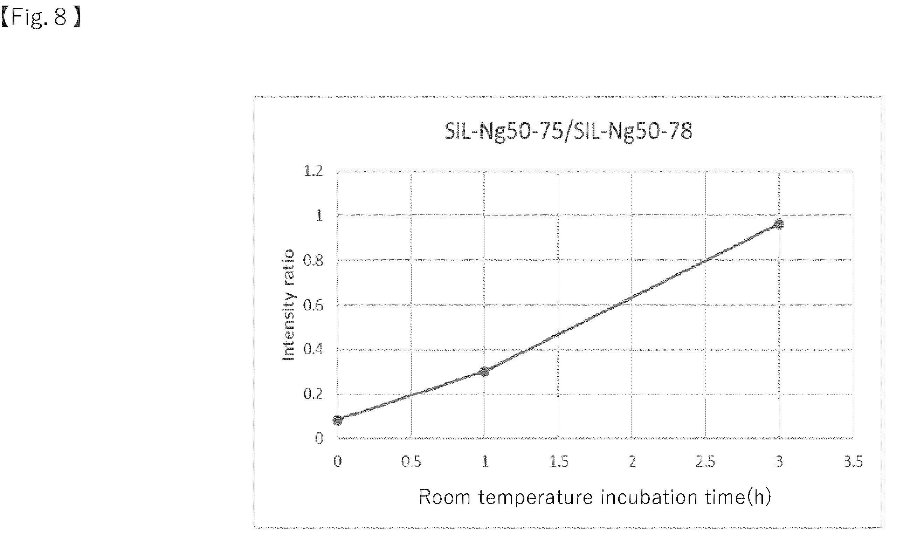

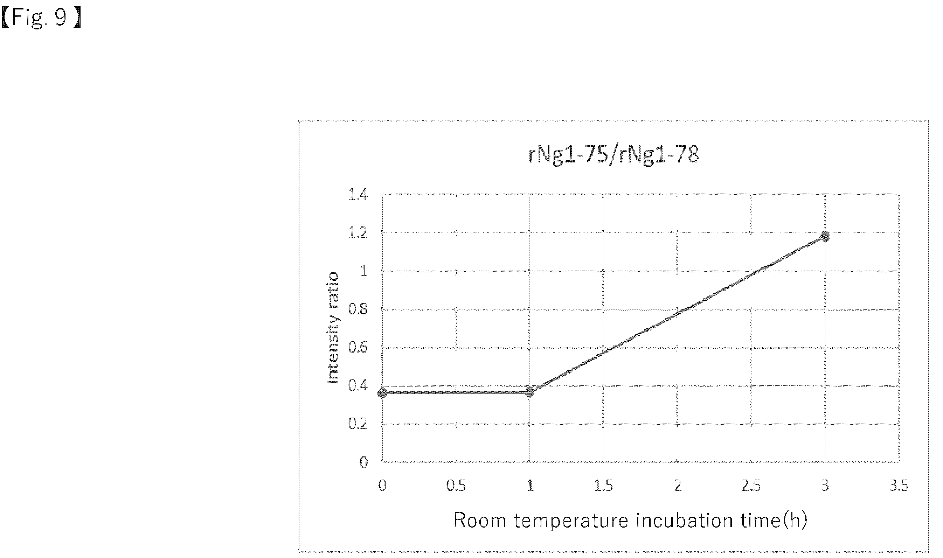

These results are illustrated in FIGS. 7 to 9. Specifically, in order to verify that Ng-related peptides collected from a living body is degraded, FIG. 7 illustrates a mass spectrum for each incubation time when a sample solution containing an Ng-related peptide and containing no DMSO is used, and FIG. 8 (SIL-Ng50-75/SIL-Ng50-78) and FIG. 9 (rNg1-75/rNg1-78) illustrates graphs showing the relationship between the intensity ratio of a degraded peptide to a peptide to be degraded and the incubation time.

### <Consideration 1>

### (1) Degradation of Ng in plasma without addition of DMSO

From FIG. 7, the peak intensity of SIL-Ng50-75 and the peak intensity of rNg1-75 increase with increasing the incubation time, and from FIGS. 8 and 9, the ratio of SIL-Ng50-75 to intact SIL-Ng50-78 and the ratio of rNg1-75 to intact rNg1-78 increase. From this, it can be seen that cleavage occurs at the C-terminal side of Ng75 over time due to the influence of protease in plasma.

### (2) Effect of suppressing Ng degradation by addition of DMSO

FIGS. 1 to 6 are analysis results after incubation for 3 hours in a state of a sample solution to which DMSO is added. From FIGS. 1 and 2, the peak of SIL-Ng50-75 and the peak of rNg1-75 are almost the same as the noise peak from the range of DMSO concentration of 5.0% or more. In addition, from FIGS. 3 to 6, the ratio of SIL-Ng50-75 to intact SIL-Ng50-78 and the ratio of rNg1-75 to intact rNg1-78 decrease as the DMSO concentration increases, and are sufficiently low values particularly in the range where the DMSO concentration is 5.0% or more. From this, it can be seen that when the DMSO concentration is 5.0% or more, the effect of suppressing Ng degradation is sufficiently exhibited. In addition, since the amounts of intact SIL-Ng50-78 and rNg1-78 become stable (constant) over time, it is also found that, in the mass spectrometry results, variations in these analysis results can be suppressed for measurement samples obtained from the same biological sample.

From FIG. 6, when the DMSO concentration is 25.0% or more, the ratio of rNg1-75 to intact rNg1-78 tends to increase. This is presumed to be because the excessive addition of DMSO changed the steric structure of rNg1-78 to decrease antigen-antibody reactivity, resulting in a decrease in the peak intensity of rNg1-78.

### <Example 2>

A sample solution containing Ng-related peptides was prepared and incubated at room temperature for 3 hours in the same manner as in Example 1 except that acetone, dimethylformamide, ACN (acetonitrile), 2-propanol, or ethanol was mixed instead of DMSO, and the final concentration was set to 10.0%. Subsequently, a purification step and a detection step were performed in the same manner as in Example 1.

### <Comparative Example 2>

A sample solution containing Ng-related peptides was prepared and incubated at room temperature for 3 hours in the same manner as in Example 1 except that methanol or toluene was mixed instead of DMSO, and the final concentration was set to 10.0%. Subsequently, a purification step and a detection step were performed in the same manner as in Example 1.

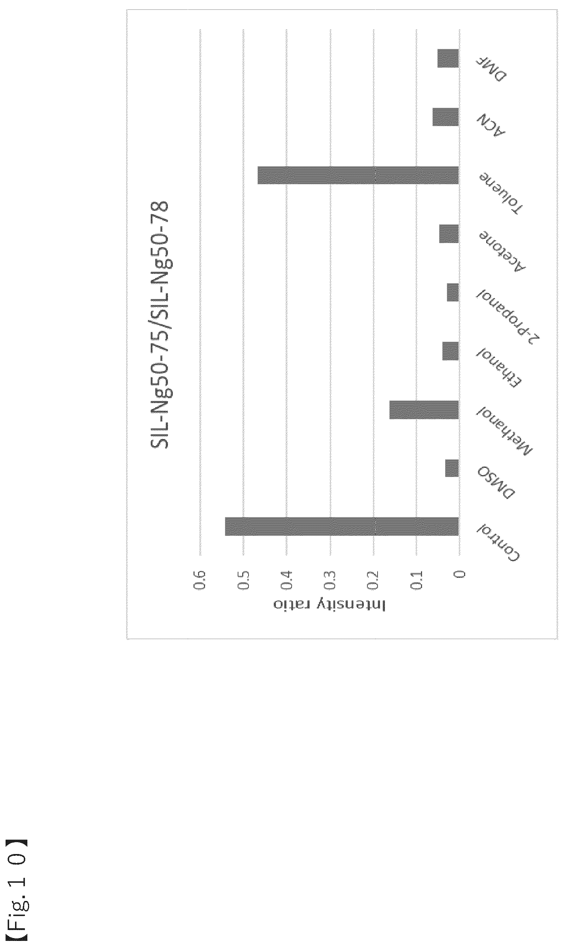

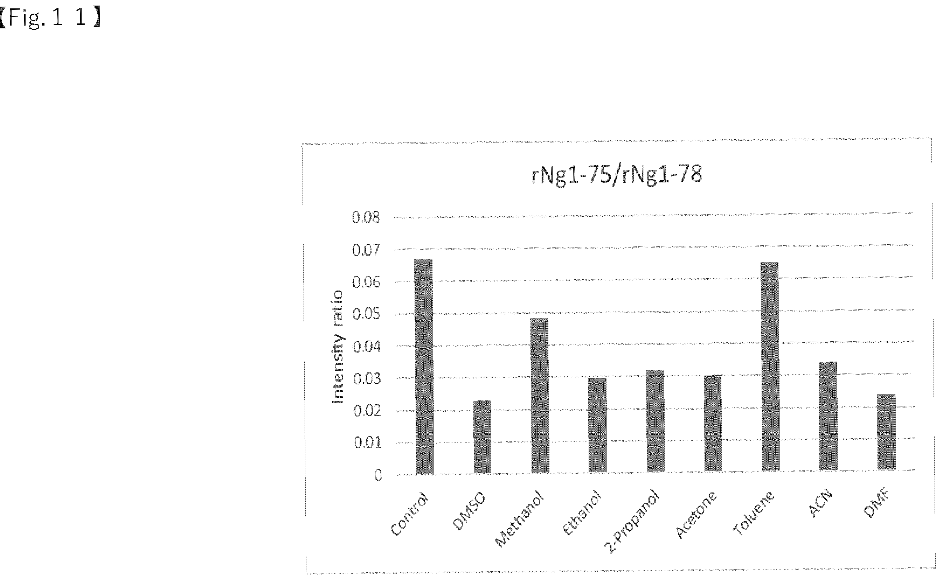

A graph showing the intensity ratio of the degraded peptide to the peptide to be degraded at this time is illustrated in FIG. 10 (SIL-Ng50-75/SIL-Ng50-78) and FIG. 11 (rNg1-75/rNg1-78). The relative polarities of the organic solvents used in Examples and Comparative Examples are shown in Table 2.

**[Table 2]**

| Organic solvent | Relative polarity |
|---|---|
| Water | 1 |
| Methanol | 0.762 |
| Ethanol | 0.654 |
| 2-Propanol | 0.546 |
| ACN | 0.460 |
| DMSO | 0.444 |
| Dimethylformamide | 0.386 |
| Acetone | 0.355 |
| Toluene | 0.099 |

### <Consideration 2>

From FIG. 10 and FIG. 11, it was found that acetone, dimethylformamide, ACN, 2-propanol, and ethanol had an effect of suppressing Ng degradation similarly to DMSO. Incidentally, it is presumed that (1) a nonpolar molecule hydrophobically interacts with an Ng-degrading enzyme in the plasma to block the hydrophobic interaction supporting a steric structure of the Ng-degrading enzyme to denature the Ng-degrading enzyme, and on the other hand, (2) a polar group of the organic solvent forms a hydrogen bond with the polar group of the Ng-degrading enzyme to denature the Ng-degrading enzyme, and from the balance between polarity and non-polarity according to (1) and (2), an organic solvent having a relative polarity (0.200 or more and 0.700 or less) within a certain range is considered to particularly effectively denature the Ng-degrading enzyme to reduce the Ng-degrading action, but the present invention is not limited to these presumed mechanisms.

### <Example 3>

A sample solution containing Ng-related peptides was prepared in the same manner as in Example 1 except that ACN was mixed instead of DMSO, and the final concentration was 0 to 30.0 (v/v%) (specifically, 0%, 0.05%, 0.25%, 0.5%, 2.5%, 5.0%, 10.0%, 15.0%, 20.0%, 25.0%, 30.0%). These were then incubated at room temperature for 3 hours. Subsequently, a purification step and a detection step were performed in the same manner as in Example 1.

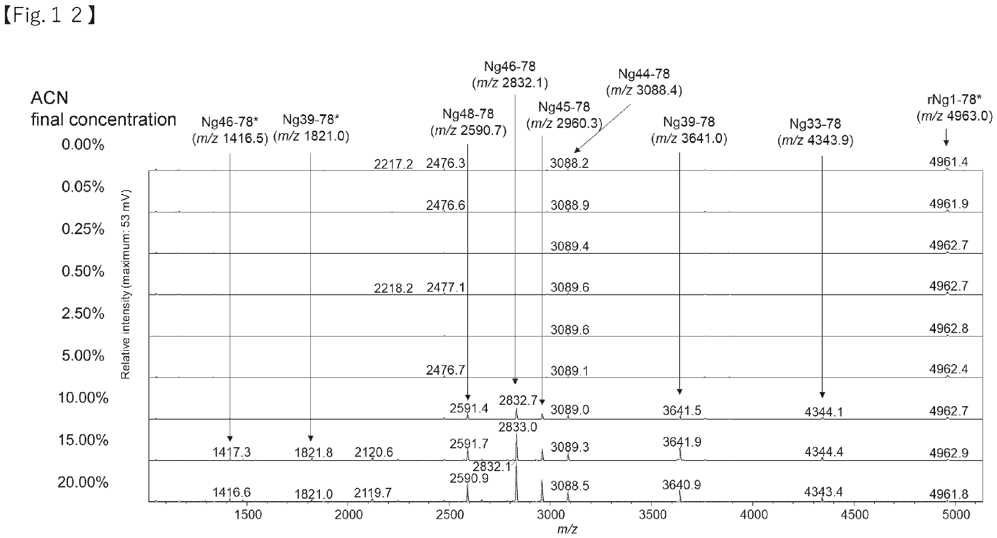

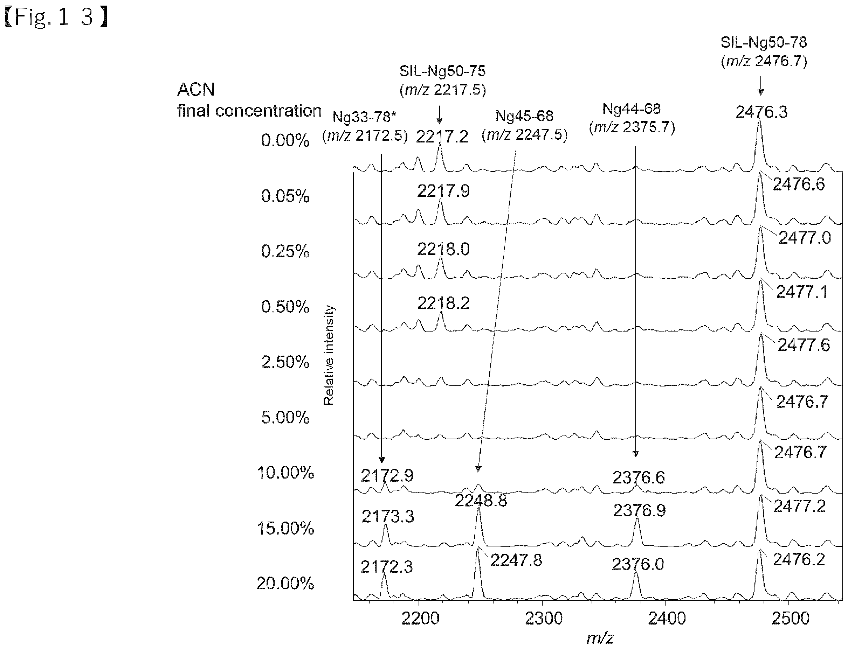

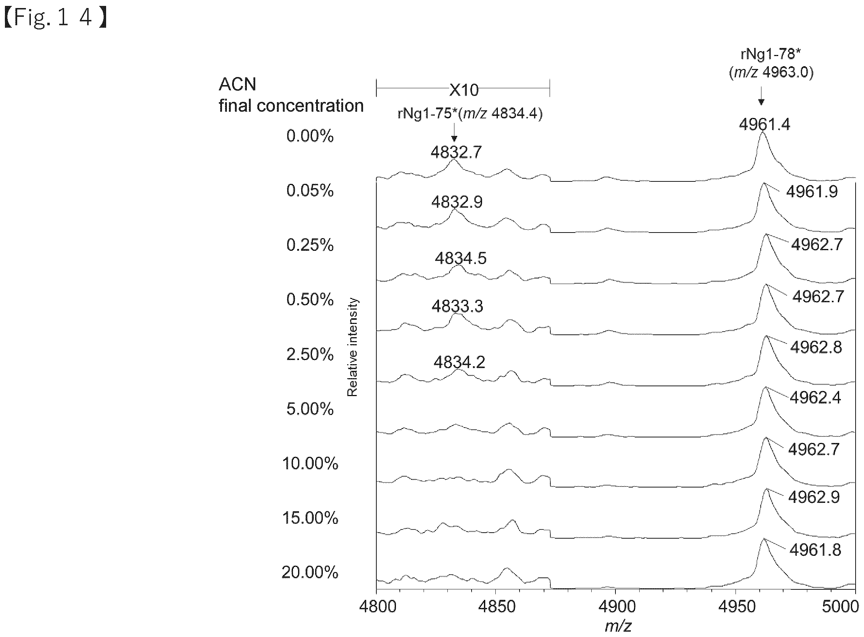

The mass spectrum results up to a final concentration of 0 to 20% at this time are illustrated in FIGS. 12 to 14. Table 3 shows the Ng-related peptides detected by the mass spectrum. In the table, * represents Theoretical average m/z of the divalent ion peak.

**[Table 3]**

| Peptide Name | Sequence | Theoretical average m/z | SEQ No. |
|---|---|---|---|
| Ng48-78 | SGERGRKGPGPGG PGGAGVARGGAGGGPSGD | 2590.7 | 5 |
| Ng46-78* | IKSGERGRKGPGPGGPGGAGVARGGAGGGPSGD | 1416.5* | 6 |
| Ng46-78 | IKSGERGRKGPGPGGPGGAGVARGGAGGGPSGD | 2832.1 | 6 |
| Ng45-78 | KIKSGERGRKGPGPGGPGGAGVARGGAGGGPSGD | 2960.3 | 7 |
| Ng44-78 | KKIKSGERGRKGPGPGGPGGAGVARGGAGGGPSGD | 3088.4 | 8 |
| Ng39-78* | GHMARKKIKSGERGRKGPGPGGPGGAGVARGGAGGGPSGD | 1821.0* | 9 |
| Ng39-78 | GHMARKKIKSGERGRKGPGPGGPGGAGVARGGAGGGPSGD | 3641 | 9 |
| Ng33-78* | IQASFRGH MARKKIKSGERGRKGPGPGGPGGAGVARGGAGGGPSGD | 2172.5* | 10 |
| Ng33-78 | IQASFRGH MARKKIKSGERGRKGPGPGGPGGAGVARGGAGGGPSGD | 4343.9 | 10 |
| Ng45-68 | KIKSGERGRKGPGPGGPGGAGVAR | 2247.5 | 11 |
| Ng44-68 | KKIKSGERGRKGPGPGGPGGAGVAR | 2375.7 | 12 |
| SIL-Ng50-75 | ERGRKGPGPGGPGGAGVARGGAGGGP | 2217.5 | 1 |
| SIL-Ng50-78 | ERGRKGPGPGGPGGAGVARGGAGGGPSGD | 2476.7 | 2 |
| rNg1-75* | GSSHHHHHHSSGLVPRGSHMGSMDCCTENACSKPDDDILDIPLDDPGAN AAAAKIQASFRGHMARKKIKSGERGRKGPGPGGPGGAGVARGGAGGGP | 4834.4 * | 3 |
| rNg1-78* | GSSHHHHHHSSGLVPRGSHMGSMDCCTENACSKPDDDILDIPLDDPGAN AAAAKIQASFRGHMARKKIKSGERGRKGPGPGGPGGAGVARGGAGGGPSGD | 4963.0 * | 4 |

### <Consideration 3>

From FIGS. 12 to 14, when the ACN concentration is 10.0 to 20.0%, a peak of a specific Ng peptides (Ng33-78, Ng45-68, Ng45-68, Ng48-78, Ng46-78, Ng45-78, Ng39-78, Ng33-78) were clearly observed, so that the signal intensity is increased and the sensitivity is improved. Furthermore, when the ACN concentration is 15.0 to 20.0%, in addition to the above, two divalent ion peaks of Ng46-78 and Ng39-78 are also observed, further improving the sensitivity. On the other hand, when the ACN concentration was 5.0% or less and 25.0% or more, the peak was not clearly observed. Also in the mass spectrum which is the analysis result of the organic solvent (DMSO, acetone, dimethylformamide, ACN, 2-propanol, ethanol, methanol, toluene) other than ACN, the peak was not clearly observed.

Regarding the sensitivity improvement of the Ng-related peptides by the addition of ACN, it is considered that ACN has a function of weakening hydrophobic interaction between proteins and peptides, and thus the Ng-related peptides adsorbed to the protein in plasma is easily released, and the antibody easily accesses the epitope, but the present invention is not limited to these presumed mechanisms.

## Claims

1. A method for preparing a sample solution containing a neurogranin-related peptide, the method comprising
mixing a biological sample containing a neurogranin-related peptide with an organic solvent having a relative polarity of 0.200 or more and 0.700 or less to prepare a sample solution having a final concentration of the organic solvent of 5.0 (v/v)% or more.

2. The preparation method according to claim 1, wherein the final concentration of the organic solvent is 5.0 (v/v)% or more and 20.0 (v/v)% or less.

3. The preparation method according to claim 1, wherein the biological sample is blood.

4. The preparation method according to claim 1, wherein a buffer is further mixed with the biological sample.

5. A method for analyzing a neurogranin-related peptide, the method comprising:
a preparation step of performing the preparation method according to any one of claims 1 to 4; and
a measurement step of performing mass spectrometry, liquid chromatography, immunoassay, or surface plasmon resonance using the sample solution.

6. The analysis method according to claim 5, wherein
the measurement step includes:
(a) a binding step of bringing the sample solution into contact with a carrier to obtain a conjugate in which the neurogranin-related peptide is bound to the carrier;
(b) a washing step of washing the conjugate using a washing solution;
(c) an elution step of bringing the conjugate into contact with an acidic solution to obtain an eluate in which the neurogranin-related peptide is eluted in the acidic solution;
and
(d) a detection step of detecting the neurogranin-related peptide in the eluate by mass spectrometry.

7. The analysis method according to claim 6, wherein an ionization method of the mass spectrometry is a matrix-assisted laser desorption/ionization method.
